Europäisches Patentamt

European Patent Office

Office européen des brevets

(19)

(11) Publication number: **0 012 402**
**B1**

(12) **EUROPEAN PATENT SPECIFICATION**

(45) Date of publication of patent specification: **18.04.84**

(21) Application number: **79105016.4**

(22) Date of filing: **10.12.79**

(51) Int. Cl.³: **C 09 J 3/14, C 08 L 33/26,**
**C 08 L 41/00, A 61 N 1/04**

(54) **Tape electrode.**

(30) Priority: **11.12.78 US 968489**

(43) Date of publication of application:
**25.06.80 Bulletin 80/13**

(45) Publication of the grant of the patent:
**18.04.84 Bulletin 84/16**

(84) Designated Contracting States:
**DE GB NL SE**

(56) References cited:
**FR - A - 2 138 752**
**US - A - 4 008 721**
**US - A - 4 066 078**
**US - A - 4 078 568**

(73) Proprietor: **MEDTRONIC, INC.**
**3055 Old Highway Eight P.O. Box 1453**
**Minneapolis Minnesota 55440 (US)**

(72) Inventor: **Cahalan, Patrick T.**
**11833 Jersey Circle**
**Champlin, MN 55316 (US)**
Inventor: **Coury, Arthur J.**
**2225 Hillside Avenue**
**St. Paul, MN 55108 (US)**

(74) Representative: **Schwan, Gerhard, Dipl.-Ing**
**Elfenstrasse 32**
**D-8000 München 83 (DE)**

Courier Press, Leamington Spa, England.

# 0 012 402

## Tape electrode

Background of prior art

In its preferred embodiments this invention is directed to medical electrodes for application to the skin. Skin electrodes are of varying types and may be used either as transmission electrodes or sensing electrodes. A wide variety of design configurations have been provided in the past for these kinds of electrodes. A desirable skin electrode is one which maintains good electrical contact with the skin and is free of localized current "hot spots". For example, a prior art electrode utilizing karaya gum tends to creep on use and flatten out exposing the skin to possible direct contact with the current distribution member or lead wire. US—A—4066078 describes electrodes for use in medical applications, comprising an adhesive electrically conductive member and electrical contact means connected to the adhesive member.

It is an object of this invention to provide electrodes in which the component contacting the skin possesses superior adhesive properties. The adhesive properties are not appreciably affected by skin moisture, allowing the electrodes to be used for several days at a time. It is also substantially homogeneous and creep resistant and is thus able to avoid the development of "hot spots". The skin contacting component is also inherently electrically conductive, not requiring electrically conductive additives.

It is a further object of this invention to provide electrodes having desirable elastic properties and wherein the skin-contacting component does not foster the growth of bacteria or other micro-organisms between the electrode and the skin.

Accordingly, an electrode of this invention comprises an electrode including an adhesive, electrically conductive member (12) and electrical contact means (14) connected to the adhesive member, characterized in that the adhesive member (12) is skin-contacting and comprises as essential components thereof a polymer consisting of polymerized 2-acrylamido-2-methylpropanesulfonic acid, its salts, copolymers of the acid, copolymers of the salts of the acid, and mixtures thereof, and a component consisting of water and/or alcohols and mixtures thereof, sufficient relative amounts of polymer and the second component being included to provide adhesiveness and flexibility to the adhesive member. Other constituents may optionally be included in the mixture and various components such as backing supports and differing electrical lead arrangements may be used.

Brief description of the drawings

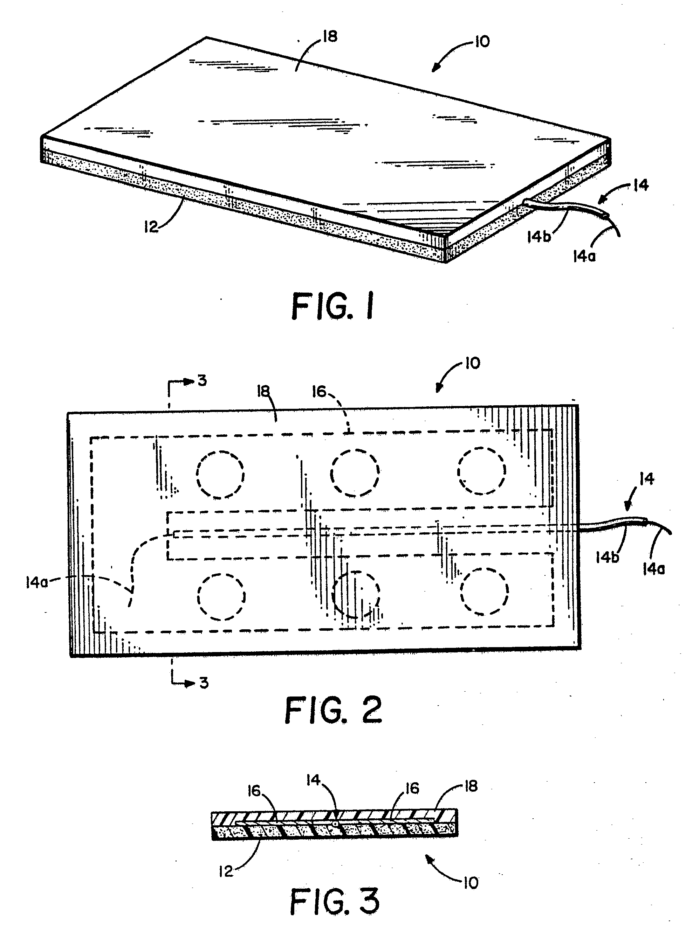

Figure 1 is a schematic perspective view of an electrode according to the invention.
Figure 2 is an elevational view of the electrode of Figure 1.
Figure 3 is a cross-sectional view taken along line 3—3 of Figure 2.

Detailed description of the invention

As previously pointed out, this invention particularly lends itself to medical electrodes of varying shapes and configurations. For exemplary purposes herein, a skin electrode 10 is shown in one of the more common rectangular configurations.

Electrode 10 includes flexible, adhesive and conductive member 12 for contacting the skin. An electrical lead 14, including a conductive member 14a and an insulating sheath or covering 14b, electrically contacts member 12.

In its most preferred form, as shown in the Figures, the electrode also includes an electrical current distribution member 16 which also electrically contacts electrical conductor 14a and member 12. In this configuration, member 12 is referred to as a substrate. Current distribution member 16 is preferably formed of a metallic foil, such as stainless steel foil, which is readily available in very thin configuration or form such as 25 $\mu$m. Such a foil may be included in the electrode without having any substantial effect on its flexibility. Due to the adhesive nature of the substrate 12, the foil readily adheres thereto. A separate adhesive such as is described below may be used for this purpose and in some instances may be preferred. Other forms of distribution member 16 may be used, such as wire mesh or conductive cloth.

The preferred embodiment of the electrode as shown in the Figures also includes a support or backing 18, the chief purpose of which is to provide a protective and supportive member for the electrode. A preferred backing material is polyethylene foam. One such material is commercially available from Fasson, Inc., a division of Avery International of Paynesville, Ohio, under the trade designation MED 416. The material is a 64 kg/m³ (four pound) density cross-linked polyethylene foam coated with a tacky adhesive material of an acrylic type. The foam is 1.59 mm thick. However, various thicknesses may be used. The foam need not be coated with the adhesive since it will in most instances, readily adhere to substrate 12 which, as previously pointed out, is adhesive itself. However, the preferred embodiment makes use of the adhesive coated foam, the stainless steel foil current distribution member 16 and the substrate member 12. The substrate member may be of various thicknesses, 0.6 to 6.4 mm being preferred. Greater thicknesses may be used as dimension is not critical as long as resistance is not excessive for the particular use involved.

2

In operation and use, electrode 10 is applied with conductive substrate 12 in direct contact with the skin. The adhesive properties of substrate 12 eliminate the need for separate adhesive tape or any other separate securing measures to hold electrode 10 in continuous contact with the skin. Upon prolonged exposure or use, the substrate may be wiped with water or alcohol to increase its adhesiveness. Electrical signals either to or from the skin are conducted through substrate member 12 and the electrical lead means, such as the current distribution member 16 and wire 14, 14 shown in the Figures.

The composition of conductive member 12 is unique to the invention. As previously stated, it includes polymerized 2-acrylamido-2-methylpropanesulfonic acid or a soluble salt thereof mixed with water and/or an alcohol, preferably glycerol or propylene glycol, in flexible sheet-like form. Copolymers of it may also be used and it may be blended with additional polymeric thickeners and conductivity improving constituents. Other alcohols may be used too. The components are provided in such relative amounts as to form a flexible, self-supporting material which has adhesive properties and is electrically conductive.

The 2-acrylamido-2-methylpropanesulfonic acid or its soluble salt may be incorporated into the mixture in the monomer form and then polymerized. Alternatively, the polymerized acid or its soluble polymerized salt may be incorporated into the mixture directly. Both approaches are illustrated in the examples below.

In the principal preferred embodiments, the member 12 is a sheet of polymerized material formed from 2-acrylamido-2-methylpropanesulfonic acid monomer purchased from The Lubrizol Corporation and sold under the tradename AMPS, a registered trademark. The monomer upon being dissolved in water, is polymerized to attain a sheet-like form which is flexible, conductive and adhesive. It is preferred that the monomer be "refined" grade as per the Lubrizol "Process for the Manufacture of Refined AMPS" dated December 14, 1976. Briefly, refined monomer is made by dissolving reaction grade monomer in methanol and recrystalllizing it. Examples 1—9 are of this type.

The term "polymer" as used hereinbelow refers to polymers of 2-acrylamido-2-methylpropanesulfonic acid or its soluble salts and, where appropriate, it is further characterized by "acid" or "salt" for additional specificity. The term "monomer" herein refers to the monomeric form of the compounds.

Example 1

50 g. of the commercially available acid monomer were dissolved in 50 g. of distilled water. The mixture was purged with nitrogen for about 10 minutes before adding the initiators. Initiator was added. The Lubrizol Corp. recommends ferrous sulfate and hydrogen peroxide in small amounts e.g., .01 g. ferrous sulfate heptahydrate and .25 g. hydrogen peroxide in a .05% solution, which was used in this example. Upon addition of the initiator, the mixture was poured into a tray in an enclosed nitrogen atmosphere to form a rectangular sheet having a thickness of about 3.2 mm. 6.4 mm thick sheets have also been prepared in this way. After pouring, the mixture rapidly gelled to a flexible material with adhesive conductive qualities.

Example 2

25 g. of the acid monomer were dissolved in 25 ml. of deionized water. 4.24 g. of ammonium hydroxide were then dissolved in this mixture. The solution was purged with nitrogen for about 10 minutes and polymerization initiator was then added. The initiator was 1 ml. of a .38 g./100 ml. solution of potassium bisulfite, 1 ml. of a .38 g./100 ml. solution of potassium persulfate and 1 ml. of a .24 g./100 ml. solution of ferrous sulfate heptahydrate. 15 seconds was allowed for mixing then the mixture was poured into a mold under a nitrogen atmosphere to form the sheet material. The mixture rapidly gelled as in Example 1.

Example 3

Same as Example 2 except 4.50 g. of lithium carbonate were substituted for the ammonium hydroxide. The resistance of the sheet was 7.5 kilo ohms for a sheet sample 38.1×25.4×3.2 mm.

Example 4

Same as Example 2 except 8.36 g. of potassium carbonate were substituted for the ammonium hydroxide. The resistance of the sheet was 2.1 kilo ohms for a sheet sample 38.1×25.4×3.2 mm.

Example 5

Same as Example 2 except 10.16 g. of sodium bicarbonate were substituted for the ammonium hydroxide. The resistance of the sheet was 2.1 kilo ohms for a sheet sample 38.1×25.4×3.2 mm.

Example 6

Another example of polymerizing the monomer and forming the sheet simultaneously was provided by mixing 50 g. of the monomer with 35 ml. of distilled water and 15 ml. of glycerol. The primary purpose of the addition of an alcohol such as glycerol is to retard drying of the polymerized sheet. The mixture was purged with nitrogen by bubbling about 10 minutes to substantially remove

3

**0 012 402**

oxygen. Initiator in the form of 1 ml. of a .15% hydrogen peroxide solution was added. The mixture was then poured onto release paper (a silicone coated paper) under a nitrogen atmosphere and it very quickly gelled to be self-supporting. It was then placed in an oven at 50°C for two hours to remove surface moisture. When placed in the oven overnight at 50°C, the material lost substantial tackiness. However, the surface of the material which was against the release paper retained good adhesive properties.

Example 7

The same procedure was used as in Example 6 except that 10 ml. of glycerol and 40 ml. of water were used. The resulting sheet was more firm than that of Example 6.

Example 8

Another sheet electrode was prepared as in Example 1. After polymerization, the electrode surface was wiped with glycerol and left exposed to ambient environment. Wiping was found to retard drying for at least 7 days. However, storage in sealed plastic bags was also found to be effective in preventing drying of the polymer sheet.

Example 9

Eight sheet-like pads of polymerized material were prepared as in Example 1 except the initiator used was: 1 ml. of a .38 g./100 ml solution of potassium bisulfite, 1 ml of a .38 g./100 ml solution of potassium persulfate and 1 ml of a .24 g./100 ml solution of ferrous sulfate heptahydrate. Each pad was about 114×178×3.2 mm in size. They were tested for skin irritation on both laboratory animals and humans with no primary skin irritation resulting.

The typical electrical resistivity of the polymerized sheet prepared and described in Examples 1 and 6—9 was about $3 \times 10^2$ ohm-cm. and the sheet was substantially colorless and substantially transparent in appearance.

The monomer and its salts may be polymerized in aqueous solutions by common water soluble radical initiators or redox systems. It can also be polymerized in emulsion, rather than in solution, using common vinyl polymerization techniques. The molecular weight of the polymer may be varied by changing the initiator concentration, the monomer concentration, the temperature or by the use of a chain transfer agent such as a mercaptan.

The molecular weight of the polymer formed for use herein is not critical so long as it is high enough to form a self-supporting gel. The higher the molecular weight, the easier the material is to handle and the better its performance, such as resistance to creep and dehydration.

Since the polymer itself i.e., poly-2-acrylamido-2-methylpropanesulfonic acid and many of its polymerized salts are water soluble, the substrate member may also be prepared by dissolving already formed polymer in water or other suitable solvents such as alcohols and forming sheet or film of desired thickness by compression forming or by solution casting and evaporating.

Additional constituents may be mixed with the polymer in such preparations also. Examples of such mixtures or "blends" are included below. Sheets of each were prepared and tested. The mixtures, in the amounts indicated below, were stirred together, placed between two pieces of mylar, pressed to desired thickness to form sheet or film, and dried overnight in an oven at 50°C.

Example 10

| | |
|---|---|
| Xanthan gum (as additional thickener) | 4.8 g. |
| acid polymer | 0.2 g. |
| water | 1.0 g. |
| glycerol | 7.0 g. |

flexibility—good, adhesion—good, residue*—marginal, elasticity—acceptable, compression**—excellent.

*residue—tacky material left on skin after removal of electrode.
**compression—resistance to flow with pressure.

Example 11

| | |
|---|---|
| Karaya gum (as additional thickener) | 2.4 g. |
| acid polymer | 0.1 g. |
| glycerol | 3.5 g. |
| water | 0.5 g. |

flexibility—excellent, adhesion—very good, residue—very good, elasticity—excellent, compression—excellent.

4

## 0 012 402

Example 12

| | |
|---|---|
| Polyacrylamide-grafted corn starch (as additional thickener) | 2.4 g. |
| acid polymer | 0.1 g. |
| glycerol | 3.5 g. |
| water | 0.5 g. |

Adhesion—excellent, compression—excellent. Resistivity $1.56 \times 10^4$ ohm-cm.

Example 13

| | |
|---|---|
| Hydroxypropyl Guar (as additional thickener) | 2.4 g. |
| acid polymer | 0.1 g. |
| glycerol | 3.5 g. |
| water | 0.5 g. |

Adhesion—good, compression—good, residue—very good. Resistivity $9.3 \times 10^1$ ohm-cm.

Example 14

| | |
|---|---|
| Evanol (a polyvinyl alcohol, as additional thickener) | 2.4 g. |
| acid polymer | 0.1 g. |
| glycerol | 3.5 g. |
| water | 0.5 g. |

Resistivity $6.89 \times 10^3$ ohm-cm.

Example 15

| | |
|---|---|
| Polyvinylpyrollidone (as additional thickener) | 2.5 g. |
| acid polymer | 1.5 g. |
| glycerol | 7. g. |
| Water | 1. g. |

Adhesion—very good. Resistivity $3.32 \times 10^2$ ohm-cm.

Example 16

| | |
|---|---|
| Polyvinylpyrollidone (as additional thickener) | 3. g. |
| acid polymer | 1. g. |
| glycerol | 4. g. |
| methanol | 10. g. |
| water | 1. g. |

Adhesion—very good. Other properties—average.

Example 17

| | |
|---|---|
| Polyacrylamide-grafted corn starch (as an additional thickener) | 2 g. |
| acid polymer | 2 g. |
| water | 4 g. |
| glycerol | 4 g. |

heat to 80°C and press to sheet form. Resistivity $1.56 \times 10^4$ ohm-cm.

Example 18

| | |
|---|---|
| Polyvinylpyrrolidone (as additional thickener) | 2 g. |
| acid polymer | 2 g. |
| water | 4 g. |
| glycerol | 4 g. |

heat to 80°C and press to sheet form. Resistivity $3.32 \times 10^2$ ohm-cm.

Example 19

| | |
|---|---|
| Evanol (a polyvinyl alcohol as additional thickener) | 2 g. |
| acid polymer | 2 g. |
| water | 4 g. |
| glycerol | 4 g. |

heat to 80°C and press to sheet form. Resistivity $6.89 \times 10^3$ ohm-cm.

5

Example 20

| Xanthan gum (as additional thickener) | 2 g. |
| acid polymer | 2 g. |
| water | 4 g. |
| glycerol | 4 g. |

heat to 80°C and pressed to sheet form. Resistivity 2.6×10² ohm-cm.

Example 21

| Hydroxypropyl guar (as additional thickener) | 2 g. |
| acid polymer | 2 g. |
| water | 4 g. |
| glycerol | 4 g. |

heat to 80°C and press to sheet form. Resistivity 9.39×10¹ ohm-cm.

Example 22

| Xanthan gum | 10 g. |
| polymer salt (lithium carbonate salt) | 2 g. |
| glycerol | 10 g. |
| water | 10 g. |

heat to 80°C and press to sheet form. Flexibility—acceptable, adhesion—acceptable, residue—acceptable, elasticity—acceptable, compression—acceptable.

Example 23

| Guar gum | 10 g. |
| polymer salt (lithium carbonate salt) | 3 g. |
| glycerol | 10 g. |
| water | 10 g. |

heat to 80°C and press to sheet form. Flexibility—acceptable, adhesion—acceptable, residue—acceptable, elasticity—acceptable, compression—acceptable.

The Xanthan gum used herein was Galaxy (a registered trade mark) XB Xanthan gum obtained from General Mills Chemicals as was the starch graft polymer used (SGP5025) and the hydroxypropyl guar used (HPG406). The Evanol was E. I. DuPont de Nemours and Co. grade 51—05 polyvinyl alcohol. The polyvinylpyrollidone was obtained from General Aniline and Film Corp. control No. 390.

Copolymers of the polymerized monomer may also be used as the conductive member 12. For example, copolymers have been successfully prepared with acrylamide, N-vinylpyrollidone and acrylic acid. Specific examples of copolymerization with acrylamide and with acrylic acid are as follows:

Example 24

Preparation and polymerization same as in Example 1.

| Acid monomer | 49.7 g. |
| acrylamide | 17.04 g. |
| water | 100 g. |
| potassium bisulfite (initiator) | 2 ml of .38 g./100 solution |
| potassium persulfate (initiator) | 2 ml of .38 g./100 solution |
| ferrous Sulfate heptahydrate (initiator) | 2 ml of .24 g./100 solution |

The resistance for a 25.4×38.1×3.2 mm sheet was 13 kilo ohms.

Example 25

| Acid monomer | 25 g. |
| acrylic acid | 4 g. |
| water | 25 g. |
| potassium bisulfite (initiator) | 1 ml of .38 g./100 solution |
| potassium persulfate (initiator) | 1 ml of .38 g./100 solution |
| ferrous sulfate heptahydrate (initiator) | 1 ml of .24 g./100 solution |

The resistance of a 25.4×38.1×3.2 mm sheet was 1.5 kilo ohms.

In general, the preferred relative amounts of constituents to be used in forming the skin contacting member 12 are indicated below.

# 0 012 402

| | Nominal amounts ingredients % by weight | Range of ingredients % by weight |
|---|---|---|
| Polymer/water | about 50/50 | 25—65/75—35 |
| polymer/alcohol | about 70/30 | 30—70/70—30 |
| polymer/water/alcohol | about 33/33/33 | 30—40/30—40/30—40 |

As can be seen from the examples provided herein, a wide variety of additives may be included in the conductive member 12, it only being necessary that it include in varying amounts, the essential polymer or copolymer of 2-acrylamido-2-methylpropanesulfonic acid or one of its soluble salts. Generally, any amount of polymer is satisfactory so long as a sufficient amount is included with water and/or alcohol to provide the requisite adhesive and flexible qualities. Various thicknesses may be used as desired and any electrode configuration with or without backing support and current distribution member may be used.

## Claims

1. An electrode including an adhesive, electrically conductive member (12) and electrical contact means (14) connected to the adhesive member, characterized in that the adhesive member (12) is skin-contacting and comprises as essential components thereof a polymer consisting of polymerized 2-acrylamido-2-methylpropanesulfonic acid, its salts, copolymers of the acid, copolymers of the salts of the acid, and mixtures thereof, and a component consisting of water and/or alcohols and mixtures thereof, sufficient relative amounts of polymer and the second component being included to provide adhesiveness and flexibility to the adhesive member.

2. An electrode according to claim 1 wherein an electrically conductive current distribution member (16) is attached to the adhesive member (12).

3. An electrode according to claim 1 or 2 wherein the adhesive member (12) comprises 25 % to 65 % polymer and 75 % to 35 % water.

4. An electrode according to claim 1 or 2 wherein the adhesive member (12) comprises 30 % to 70 % polymer and 70 % to 30 % alcohol.

5. An electrode according to claim 1 or 2 wherein the adhesive member (12) comprises 30 % to 40 % polymer, 30 % to 40 % water and 30 % to 40 % alcohol.

6. An electrode according to claim 1, 2, 4 or 5 wherein the alcohol is glycerol.

7. An electrode according to claim 1, 2, 4 or 5 wherein the alcohol is propylene glycol.

8. An electrode according to any one of claims 2 to 7 including a polymer foam backing member (18) overlying the current distribution member (16).

9. An electrode according to any one of claims 1 to 8 wherein the adhesive member (12) is in sheet form, ranging in thickness from 0.6 to 6.4 millimeters.

10. An electrode according to any one of claims 1 to 9 wherein the adhesive member (12) additionally includes thickening constituents.

11. An electrode according to any one of claims 1 to 10 wherein the adhesive member (12) additionally includes conductivity improving constituents.

## Patentansprüche

1. Elektrode mit einem haftenden, elektrisch leitenden Teil (12) und einer mit dem haftenden Teil verbundenen elektrischen Kontaktanordnung (14), dadurch gekennzeichnet, daß das haftende Teil (12) als Hautkontaktteil ausgebildet ist und als. wesentliche Komponenten ein Polymer bestehend aus polymierisierter 2-Acrylamid-2-methylpropansulfonsäure, deren Salze, Kopolymeren der Säure, Kopolymeren der Salze der Säure und Gemischen davon, sowie eine Komponente bestehend aus Wasser und/oder Alkoholen und Gemischen davon aufweist, wobei ausreichende relative Mengen des Polymers und der zweiten Komponente vorgesehen sind, um dem haftenden Teil Haftungsvermögen und Flexibilität zu verleihen.

2. Elektrode nach Anspruch 1, wobei an dem haftenden Teil (12) ein elektrisch leitendes Stromverteilungsorgan (16) angebracht ist.

3. Elektrode nach Anspruch 1 oder 2, wobei das haftende Teil (12) 25% bis 65% Polymer und 75% bis 35% Wasser aufweist.

4. Elektrode nach Anspruch 1 oder 2, wobei das haftende Teil (12) 30% bis 70% Polymer und 70% bis 30% Alkohol aufweist.

7

5. Elektrode nach Anspruch 1 oder 2, wobei das haftende Teil (12) 30% bis 40% Polymer, 30% bis 40% Wasser und 30% bis 40% Alkohol aufweist.

6. Elektrode nach Anspruch 1, 2, 4 oder 5, wobei als Alkohol Glyzerol vorgesehen ist.

7. Elektrode nach Anspruch 1, 2, 4 oder 5, wobei als Alkohol Propylenglykol vorgesehen ist.

8. Elektrode nach einem der Ansprüche 2 bis 7 mit einem über dem Stromverteilungsorgan (16) liegenden rückseitigen Teil (18) aus Polymerschaum.

9. Elektrode nach einem der Ansprüche 1 bis 8, wobei das haftende Teil (12) in Blattform mit einer Dicke von 0,6 bis 6,4 mm vorliegt.

10. Elektrode nach einem der Ansprüche 1 bis 9, wobei das haftende Teil (12) zusätzlich Eindickungsmittel aufweist.

11. Elektrode nach einem der Ansprüche 1 bis 10, wobei das haftende Teil (12) zusätzlich die Leitfähigkeit verbessernde Bestandteile aufweist.

**Revendications**

1. Electrode comprenant un organe adhésif et électriquement conducteur (12) et des moyens de contact électrique (14) reliés audit organe (12), caractérisée en ce que l'organe adhésif (12) est en contact avec la peau et comporte comme composants essentiels un polymère constitué d'acide 2-Acrylamido 2-méthylpropanesulfonique polymérisé, de ses sels, des copolymères de l'acide, des copolymères des sels de l'acide, et des mélanges de ces composants, et un composant constitué d'eau et/ou d'alcool et de leurs mélanges, des quantités relatives suffisantes du polymère et du second composant étant incluses pour fournir un pouvoir adhésif et de la souplesse à l'organe adhésif.

2. Electrode selon la revendication 1, caractérisée en ce qu'une pièce de distribution de courant (16) électriquement conductrice est reliée à l'organe adhésif (12).

3. Electrode selon la revendication 1 ou 2, caractérisée en ce que l'organe adhésif (12) comprend de 25 % à 65 % de polymère et de 75 % à 35 % d'eau.

4. Electrode selon la revendication 1 ou 2, caractérisée en ce que l'organe adhésif (12) comprend de 30 % à 70 % de polymère et de 70 % à 30 % d'alcool.

5. Electrode selon la revendication 1 ou 2, caractérisée en ce que l'organe adhésif (12) comprend de 30 % à 40 % de polymère, de 30 % à 40 % d'eau et de 30 % à 40 % d'alcool.

6. Electrode selon la revendication 1, 2, 4 ou 5, caractérisée en ce que l'alcool est du glycérol.

7. Electrode selon la revendication 1, 2, 4 ou 5, caractérisée en ce que l'alcool est du propyléne glycol.

8. Electrode selon l'une quelconque des revendications 2 à 7, caractérisée en ce qu'elle comprend une pièce support (18) en mousse de polymère recouvrant la pièce de distribution de courant (16).

9. Electrode selon l'une quelconque des revendications 1 à 8, caractérisée en ce que l'organe adhésif (12) a la forme d'une feuille, dont l'épaisseur se situe dans la gamme de 0,6 mm à 6,4 mm.

10. Electrode selon l'une quelconque des revendications 1 à 9, caractérisée en ce que l'organe adhésif (12) comprend en plus des composants épaississeurs.

11. Electrode selon l'une quelconque des revendications 1 à 10, caractérisée en ce que l'organe adhésif (12) comprend additionnellement, des composants améliorant la conductivité.

0 012 402

FIG. 1

FIG. 2

FIG. 3

1